# EUROPÄISCHE PATENTSCHRIFT

(11) **EP 0 614 891 B1**
(45) Veröffentlichungstag und Bekanntmachung des Hinweises auf die Patenterteilung: **30.12.1998**
(21) Anmeldenummer: 94103714.5
(22) Anmeldetag: 10.03.1994
(51) Int. Cl.: C07D 233/68

(54) **Verfahren zur Herstellung von 2-substituierten 5-Chlorimidazol-4-carbaldehyden**
Process for the preparation of 2-substituted 5-chlorimidazol-4-aldehydes
Procédé pour la préparation des 5-chlorimidazole-4-aldehydes substitués sur la position 2

(30) Priorität: 12.03.1993 CH 748/93
(43) Veröffentlichungstag der Anmeldung: 14.09.1994
(73) Patentinhaber: LONZA AG, CH-3945 Gampel/Wallis (CH)
(72) Erfinder: Griffiths, Gareth, Dr., Visp (Kanton Wallis) (CH); Imwinkelried, René, Dr., Brig-Glis (Kanton Wallis) (CH); Gosteli, Jacques, Dr., Basel (CH)
(74) Vertreter: Weinhold, Peter, Dr.

(56) Entgegenhaltungen:
- EP-A- 0 028 834
- EP-A- 0 253 310
- EP-A- 0 365 030
- EP-A- 0 429 257
- EP-A- 0 479 479
- EP-A- 0 505 098
- EP-A- 0 579 212
- DE-A- 2 804 435
- DE-A- 3 145 927
- DE-A- 3 330 192
- US-A- 3 409 606
- JOURNAL OF HETEROCYCLIC CHEMISTRY, Bd.27, Nr.3, 1990 Seiten 711 - 716 T. WATANABE ET AL.
- JOURNAL OF HETEROCYCLIC CHEMISTRY, Bd.4, 1967 Seiten 399 - 402 A.W. LUTZ ET AL.
- JOURNAL OF HETEROCYCLIC CHEMISTRY, Bd.4, 1967 Seiten 451 - 452 J.L. IMBACH ET AL.
- JOURNAL OF THE CHEMICAL SOCIETY, PERKIN TRANSACTIONS I, Nr.7, 1980 Seiten 2310 - 2315 T. BROWN ET AL.
- HETEROCYCLES, Bd.35, Nr.1, 1993 Seiten 121 - 124 D.H. BOSCHELLI ET AL.
- PATENT ABSTRACTS OF JAPAN vol. 12, no. 117 (C-487) 13. April 1988 & JP-A-62 238 383 (CHIYODA KAGAKU KENKYUSHO K.K.) 19. Oktober 1987
- BULLETIN DE LA SOCIETE CHIMIQUE DE FRANCE, Nr.3, 1971 Seiten 1040 - 1051 R. JACQUIER ET AL.
- CHEMICAL AND PHARMACEUTICAL BULLETIN, Bd.24, Nr.5, 1976 Seiten 960 - 969 K. MATSUMURA ET AL. 'Studies of Nitriles. XI. Preparation and Chemistry of Schiff Bases of ADAN ...'
- PATENT ABSTRACTS OF JAPAN vol. 16, no. 190 (C-0937) 8. Mai 1992 & JP-A-04 026 678 (NIPPON SYNTHETIC CHEM IND CO LTD) 29. Januar 1992
- Advanced Organic Chemistry, J.March, Wiley, New York, 1992

## Beschreibung

Die vorliegende Erfindung betrifft ein Verfahren zur Herstellung von 2-substituierten 5-Chlorimidazol-4-carbaldehyden der allgemeinen Formel 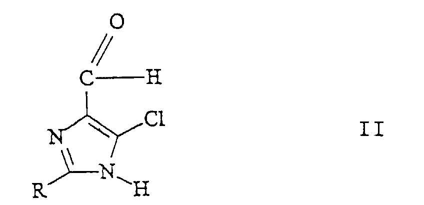 worin R eine geradkettige oder verzweigte C₁-C₆-Alkylgruppe, eine geradkettige oder verzweigte C₂-C₆-Alkenylgruppe, eine Cyclopropyl-, Cyclobutyl-, Cyclopentyl- oder Cyclohexylgruppe oder eine gegebenenfalls mit Halogen, Nitro, Amino und/oder Alkyl substituierte Benzylgruppe oder Phenylgruppe bedeutet.

Bei diesem Verfahren wird ein 2-substituiertes 4-Chlorimidazol der allgemeinen Formel 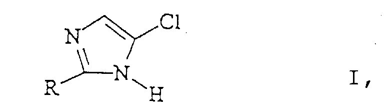 das wie in der EP-A-614 890 beschrieben hergestellt wird, mit Phosphoroxychlorid oder Phosgen in Gegenwart von N,N-Dimethylformamid zum Endprodukt der Formel II umgesetzt.

Die 2-substituierten 5-Chlorimidazol-4-carbaldehyde der allgemeinen Formel II sind wichtige Ausgangsmaterialien zur Herstellung von blutdrucksenkenden Pharmazeutika (EP-A-0 028 834, entsprechend US-A-4 355 040) oder von herbizid-wirksamen Verbindungen (DE-A 2 804 435). Zur Herstellung der 2-substituierten 5-Chlorimidazol-4-carbaldehyde sind mehrere Wege bekannt.

So beschreibt die EP-A-0 028 834 ein Verfahren, nach welchem 2-Amino-3,3-dichloracrylnitril mit einem Aldehyd zum entsprechenden Azomethinzwischenprodukt und weiter mit einem Halogenwasserstoff und Wasser zum 2-substituierten 5-Halogenimidazol-4-carbaldehyd umgesetzt wird. Experimentelle Angaben fehlen in der genannten Patentschrift. Ein großer Nachteil der Synthese besteht aber darin, daß das eingesetzte 2-Amino-3,3-dichloracrylnitril zunächst ausgehend von Dichloracetonitril durch dessen Umsetzung mit Blausäure/Natriumcyanid hergestellt werden muß. Die äußerst toxischen Reaktionsteilnehmer und die damit verbundenen sicherheitstechnischen Maßnahmen, die bereits für die Bereitstellung des Ausgangsproduktes notwendig sind, machen das Gesamtverfahren industriell untauglich.

In einer weiteren Variante offenbart die EP-A-0 028 834 ein 3-Stufen Verfahren, worin in einer ersten Stufe ein Amidinhydrochlorid mit Dihydroxyaceton mit hohem NH₃-Druck ringgeschlossen wird, der Imidazolalkohol halogeniert wird und schließlich zum Aldehyd oxidiert wird. Es hat sich gezeigt, daß für die Ringschlußreaktion Drucke von über 20 bar notwendig sind. Die Oxidation des Alkohols funktioniert ferner gemäß EP-A-0 028 834 in Gegenwart von Chromoxid. Es ist offensichtlich, daß eine Oxidation mit Schwermetalloxiden, die in der Regel nicht rezirkulierbar sind, nach heutigen ökologischen Gesichtspunkten nicht mehr verantwortbar ist.

Ähnlich beschreibt die DE-A-2 804 435 die Herstellung von 2-substituierten Imidazol-4-carbaldehyden durch Oxidation entsprechender Imidazolalkohole mit konzentrierter Salpetersäure oder aktiviertem Mangandioxid.

Die Formylierung aromatischer Hebrozyklen durch Reaktion mit disubstituierten Formamiden und Phosphoroxychlorid oder Phosgen, die sog. Vilsmeier-Reaktion, wird allgemein bei J. March, Advanced Organic Chemistry, John Wiley & Sons, New York, 4. Aufl., S. 542 (1992), beschrieben.

Substituierte Chlorimidazole als Ausgangsprodukte für die Herstellung von Angiotensin II-Antagonisten werden in der EP-A-0 505 098 beschrieben. Die EP-A-0 429 257 beschreibt halogensubstituierte Imidazole als Ausgangsprodukte für die Herstellung von Indolderivaten, die beispielsweise zur Behandlung und Prophylaxe von Bluthochdruck eingesetzt werden können. Keine der genannten Druckschriften offenbart jedoch das erfindungsgemäße Verfahren zur Herstellung von 2-substituierten 5-Chlorimidazol-4-carbaldehyden.

Aufgabe der vorliegenden Erfindung war es, ein Verfahren zur Herstellung von 2-substituierten 5-Chlorimidazol-4-carbaldehyden ohne die oben erwähnten Nachteile bereitzustellen. Diese Aufgabe konnte durch das Verfahren gemäß Anspruch 1 gelöst werden.

Zur Herstellung der in der letzten Stufe des erfindungsgemäßen Verfahrens umgesetzten 2-substituierten 4-Chlorimidazole der allgemeinen Formel I wird wie in der EP-A-614 890 beschrieben vorgegangen. So wird in der ersten Stufe ein Glycinesterhydrohalogenid der allgemeinen Formel 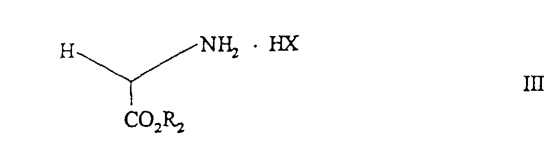 worin R₂ für eine Alkylgruppe steht und X für ein Halogenatom steht, mit einem Imidsäureester der allgemeinen Formel 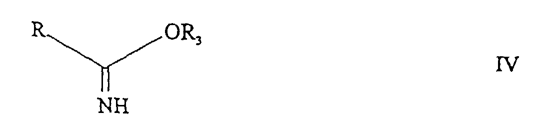 worin R die genannte Bedeutung hat und R₃ eine Alkylgruppe bedeutet, in Gegenwart einer Base zum entsprechenden 2-substituierten 3,5-Dihydroimidazol-4-on der allgemeinen Formel 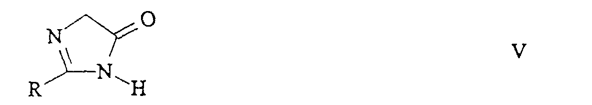 worin R die genannte Bedeutung hat, umgesetzt.

In den allgemeinen Substituenten R, R₂ und R₃ haben die angegebenen Gruppen folgende Bedeutung:

Unter einer Alkylgruppe werden geradkettige oder verzweigte C₁-C₆-Alkylgruppen, wie z.B. Methyl-, Ethyl-, n-Propyl-, Isopropyl-, n-Butyl, sec. Butyl-, tert. Butyl-, Pentyl- oder Hexylgruppen verstanden. Bevorzugte Alkylgruppe ist eine der genannten C₁-C₄-Alkylgruppen. Bevorzugt für Substituent R ist die n-Butylgruppe.

Unter einer Alkenylgruppe wird eine geradkettige oder verzweigte C₂-C₆-Alkenylgruppe, wie z.B. 1-Propenyl, 2-Propenyl, 1-Butenyl, 2-Butenyl, 3-Butenyl, Pentenyl und seine Isomeren oder Hexenyl und seine Isomeren, verstanden. Für R ist die 2- oder 3-Butenyl-Gruppe bevorzugt.

Als Vertreter von Cycloalkylgruppen seien die Cyclopropyl-, die Cyclobutyl-, die Cyclopentyl- oder die Cyclohexylgruppe genannt.

Sowohl die Benzyl- als auch die Phenylgruppe kann Substituenten, wie die genannten Alkylgruppen, Halogenatome, Nitrogruppen oder Aminogruppen, enthalten.

Unter der Bezeichnung Halogen wird zweckmässig Chlor, Brom oder Jod, vorzugsweise Chlor, verstanden.
Zweckmässig wird so vorgegangen, dass das Glycinesterhydrohalogenid der allgemeinen Formel III in Gegenwart einer Base, zweckmässig bei einem pH-Wert von 7 bis 12, vorzugsweise von 9 bis 11, mit dem Imidsäureester der allgemeinen Formel IV reagiert wird.

Die Glycinesterhydrohalogenide der allgemeinen Formel III sind im Handel erhältliche stabile Verbindungen.

Geeignete Basen sind die Alkalihydroxide, wie z.B. Natriumhydroxid oder Kaliumhydroxid oder Alkalialkoholate, wie z.B. Natrium- oder Kaliummethylat, -ethylat oder -tert.-butylat.

Vorteilhaft liegt die Base in einem geeigneten Lösungsmittel gelöst vor. Besonders geeignet sind aliphatische Alkohole wie Methanol oder Ethanol. Der Imidsäureester wird zweckmässig ebenfalls in Form einer Lösung in einem inerten Lösungsmittel zugegeben. In der Regel eignen sich hierzu aromatische Lösungsmittel , wie z.B. Toluol oder Chlorbenzol, oder die genannten aliphatischen Alkohole besonders gut.
Von Vorteil erfolgt die Umsetzung der Reaktionsteilnehmer Glycinesterhydrohalogenid, Imidsäureester und Base im stöchiometrischen Verhältnis 1:1:1.

Die Umsetzungstemperatur rangiert zweckmässig im Bereich von -20°C bis 50°C, vorzugsweise bei 0° bis 25°C.

Nach einer Umsetzungszeit von wenigen Stunden kann das entsprechende 3,5-Dihydroimidazol-4-on der allgemeinen Formel V auf fachmännische Weise, in der Regel durch einfache Filtration, in Ausbeuten von grösser als 95% isoliert werden.

Von Vorteil wird das resultierende Reaktionsgemisch ohne Isolierung des 2-substituierten 3,5-Dihydroimidazol-4-ons für die Weiterumsetzung zum entsprechenden 4-Chlorimidazol bereitgestellt (Eintopfverfahren).

Diese erste Stufe des erfindungsgemässen Verfahrens beinhaltet eine sprunghafte Verbesserung des bekannten Verfahrens nach R. Jacquier et al.(Bull. Soc. Chim. France, 1971, 1040), welches die Umsetzung des freien Glycinesters mit einem Imidsäureethylester in Abwesenheit eines Lösungsmittels zum entsprechenden 3,5-Dihydroimidazol-4-on umfasst. Nachteilig bei diesem bekannten Verfahren ist, dass der freie Glycinester sehr instabil ist und daher für jede Umsetzung jeweils neu synthetisiert und isoliert werden muss. Gemäss dem bekannten Verfahren konnten nach einer Umsetzungszeit von 24 Std. und länger Ausbeuten von lediglich 30% bis 48% erhalten werden.

In der zweiten Stufe wird das 3,5-Dihydroimidazol-4-on der allgemeinen Formel V zum entsprechenden 4-Chlorimidazol der allgemeinen Formel I chloriert.

Zweckmässig erfolgt die Chlorierung mit Thionylchlorid oder Phosphoroxychlorid, vorteilhaft in einem Überschuss an Chlorierungsmittel von 10 bis 300% bei einer Reaktionstemperatur im Bereich zwischen 20°C und 110°C. Das Chlorierungsmittel kann dabei zugleich als Lösungsmittel dienen, so dass ein zusätzliches Lösungsmittel in der Regel nicht notwendig ist. Bevorzugt wird Phosphoroxychlorid als Chlorierungsmittel angewendet. Das resultierende 2-substituierte 4-Chlorimidazol der allgemeinen Formel I kann auf fachmännische Weise, vorteilhaft durch Extraktion, in hoher Reinheit aus dem Reaktionsgemisch isoliert werden.

Bevorzugte 2-substituierte 4-Chlorimidazole der allgemeinen Formel I sind solche, worin R die Bedeutung von n-Butyl, 2-Butenyl oder 3-Butenyl hat.

Die Umsetzung zum gewünschten 5-chlorimidazol-4-carbaldehyd der allgemeinen Formel II erfolgt erfindungsgemäss mit Phosphoroxychlorid oder Phosgen in Gegenwart von N,N-Dimethylformamid.

Zweckmässig liegt das Molverhältnis der Reaktanden 2-substituiertes 5-Chlorimidazol zu Phosphoroxychlorid oder Phosgen zu N,N-Dimethylformamid im Bereich zwischen 1:1:1 und 1:5:5, bevorzugt bei ungefähr 1:3:3.

Die Umsetzungstemperatur liegt zweckmässig zwischen 50° und 130°C.

Gegebenenfalls kann in Gegenwart eines zusätzlichen inerten Lösungsmittels ,beim Eintopfverfahren vorteilhaft im Lösungsmittel der ersten Stufe, gearbeitet werden.

Die Isolierung des resultierenden 2-substituierten 5-Chlorimidazol-4-carbaldehyds aus dem Reaktionsgemisch erfolgt auf fachmännische Weise vorteilhaft durch dessen Extraktion mit einem geeigneten Lösungsmittel.

### Beispiele:

### Herstellung von 2-n-Butyl-3,5-dihydroimidazol-4-on

Zu einer Lösung von 10,1 g (0,25 mol) Natriumhydroxid in Methanol bei 0°C wurden 31,71 g (0,25 mol) Glycinmethylesterhydrochlorid zugegeben. Nach 15 Minuten wurden zur weissen Suspension 126,5 g einer 22,8%igen Lösung von Pentanimidsäuremethylester in Chlorbenzol während 5 Minuten zugetropft. Die hellgelbe Suspension wurde 4 Std. bei Raumtemperatur gerührt und mit Chlorbenzol (100 ml) verdünnt. Das Methanol wurde bei einer Temperatur von 26°C und einem Druck von 30 - 50 mbar abdestilliert,und die orange Suspension wurde mit Methylenchlorid (100 ml) verdünnt und anschliessend filtriert. Nach Entfernung des Lösungsmittels vom Filtrat wurden 34,08 g (97%). der Titelverbindung (Gehalt >95% nach GC und ¹H-NMR) erhalten.

### Herstellung von 2-n-Butyl-5-chlor-1H-imidazol

2-n-Butyl-3,5-dihydroimidazol-4-on (14,02 g, 0,1 mol) wurde portionenweise während 15 Minuten zu POCl₃ (50 ml) bei 95°C gegeben. Die Lösung wurde 2 Std. bei 100°C erhitzt, gekühlt und auf 400 g Eis gegossen. Das Gemisch wurde mit 255 ml 30%iger Natronlauge auf pH 7 gestellt und dreimal mit je 500 ml Essigester extrahiert. Die vereinigten organischen Phasen wurden mit MgSO₄ getrocknet, filtriert und am Rotavapor® eingeengt. Nach Reinigung des Rückstands mittels Säulenchromatographie wurde die Titelverbindung (5,52 g, 34,7%) in hoher Reinheit (>98% nach GC und ¹H-NMR) erhalten.
Smp. 85° - 87°C
¹H-NMR (CDCl₃) δ 0,91 (3H, t, J = 7,5 Hz),
1,36 (2H, sextett, J = 7,5 Hz),
1,68 (2H, q, J = 7,5 Hz),
2,70 (2H, t, J = 7,5 Hz),
6,83 (1H, s),
10,65 (1H, br s).

### Herstellung von 2-n-Butyl-5-chlorimidazol-4-carbaldehyd aus 2-n-Butyl-5-chlor-1H-imidazol

Zu einer auf 95°C erwärmten Lösung von 2-n-Butyl-5-chlor-1H-imidazol (1,60 g, 10 mmol) in POCl₃ (3,07 g, 20 mmol) und Chlorbenzol (20 ml) wurde N,N-Dimethylformamid (1,46 g, 20 mmol) zugetropft. Das Gemisch wurde während 3,5 Std. bei 98°C gerührt. Danach wurden weitere Portionen von POCl₃ (1,53 g, 10 mmol) und von N,N-Dimethylformamid (0,73 g, 10 mmol) zugetropft. Nach weiteren 2,5 Std. bei 98°C wurde das Gemisch gekühlt und auf Eis (40 g) gegossen. Nach 15 Minuten wurde das Gemisch mit 11 ml 30%iger Natronlauge auf pH 7 gestellt und dreimal mit je 100 ml Essigester extrahiert. Die vereinigten organischen Phasen wurden mit MgSO₄ getrocknet, filtriert und eingeengt. Die Titelverbindung wurde in einer Ausbeute von 1,3 g (70%) erhalten.

## Patentansprüche

1. Verfahren zur Herstellung von 2-substituierten 5-Chlorimidazol-4-carbaldehyden der allgemeinen Formel 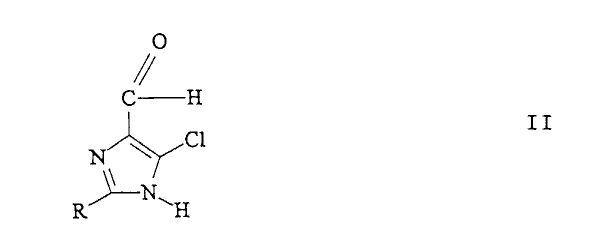 worin R eine geradkettige oder verzweigte C₁-C₆-Alkylgruppe, eine geradkettige oder verzweigte C₂-C₆-Alkenylgruppe, eine Cyclopropyl-, Cyclobutyl-, Cyclopentyl- oder Cyclohexylgruppe oder eine gegebenenfalls mit Halogen, Nitro, Amino und/oder Alkyl substituierte Benzylgruppe oder Phenylgruppe bedeutet, dadurch gekennzeichnet, daß man zunächst ein Glycinesterhydrohalogenid der allgemeinen Formel 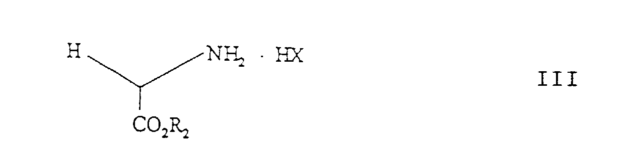 worin R₂ eine Alkylgruppe bedeutet und X für ein Halogenatom steht, mit einem Imidsäureester der allgemeinen Formel 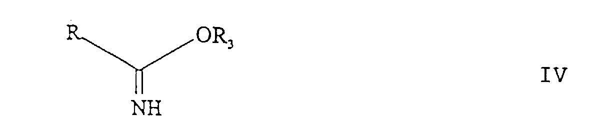 worin R die genannte Bedeutung hat und R₃ eine Alkylgruppe bedeutet, in Gegenwart einer Base zum entsprechenden 3,5-Dihydroimidazol-4-on der allgemeinen Formel 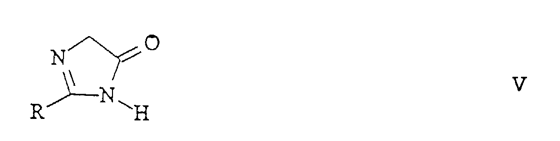 worin R die genannte Bedeutung hat, umsetzt, dieses anschließend zu einem 2-substituierten 4-Chlorimidazol der allgemeinen Formel 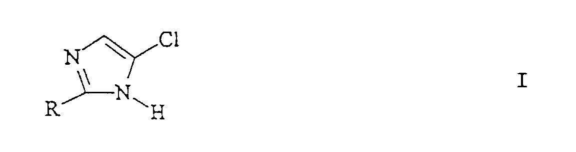 worin R die genannte Bedeutung hat, chloriert, und letzteres schließlich mit Phosphoroxychlorid oder Phosgen in Gegenwart von N,N-Dimethylformamid zum Endprodukt der Formel II umsetzt.

2. Verfahren nach Anspruch 1, worin als Base ein Alkalihydroxid oder ein Alkalialkoholat verwendet wird.

3. Verfahren nach einem der Ansprüche 1 oder 2, worin die Umsetzung zum 3,5-Dihydroimidazol-4-on der Formel V in einem pH-Bereich zwischen 7 und 12 durchgeführt wird.

4. Verfahren nach einem der Ansprüche 1 bis 3, worin die Reaktionsteilnehmer für die Umsetzung zum 3,5-Dihydroimidazol-4-on der Formel V, nämlich Glycinesterhydrohalogenid, Imidsäureester und Base, im stöchiometrischen Verhältnis von 1:1:1 umgesetzt werden.

5. Verfahren nach einem der Ansprüche 1 bis 4, worin die Reaktionstemperatur für die Umsetzung zum 3,5-Dihydroimidazol-4-on der Formel V zwischen -20°C und 50°C liegt.

6. Verfahren nach einem der Ansprüche 1 bis 5, worin die Chlorierung zum Chlorimidazol der Formel I mit Thionylchlorid oder Phosphoroxychlorid durchgeführt wird.

7. Verfahren nach Anspruch 6, worin die Chlorierung mit Phosphoroxychlorid bei einer Umsetzungstemperatur zwischen 20°C und 110°C durchgeführt wird.

8. Verfahren nach einem der Ansprüche 1 bis 7, worin die Reaktionsteilnehmer der letzten Stufe, nämlich das 2-substituierte 4-Chlorimidazol der Formel I, das Phosphoroxychlorid oder das Phosgen, und das N,N-Dimethylformamid, im Molverhältnis von 1:1:1 bis 1:5:5 umgesetzt werden.

9. Verfahren nach einem der Ansprüche 1 bis 8, worin die Umsetzungstemperatur in der letzten Stufe zwischen 50°C und 130°C liegt.

## Claims

1. Process for the production of 2-substituted 5-chloroimidazole-4-carbaldehydes of the general formula 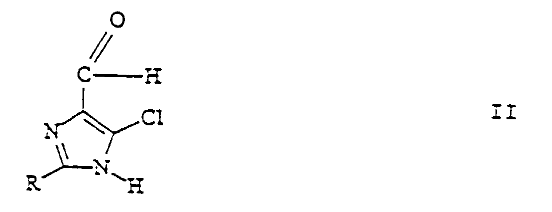 in which R means a linear or branched C₁-C₆ alkyl group, a linear or branched C₂-C₆ alkenyl group, a cyclopropyl, cyclobutyl, cyclopentyl or cyclohexyl group or a benzyl group or phenyl group optionally substituted with halogen, nitro, amino and/or alkyl, characterised in that a glycine ester hydrohalide of the general formula 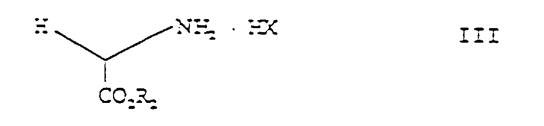 in which R₂ means an alkyl group and X denotes a halogen atom, is initially reacted with an imide acid ester of the general formula 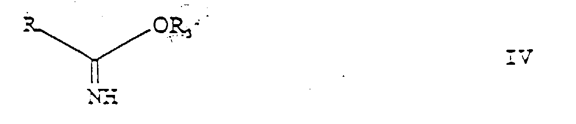 in which R has the stated meaning and R₃ means an alkyl group, in the presence of a base to yield the corresponding 3,5-dihydroimidazol-4-one of the general formula 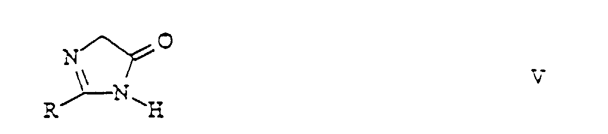 in which R has the stated meaning, this compound is then chlorinated to yield a 2-substituted 4-chloroimidazole of the general formula 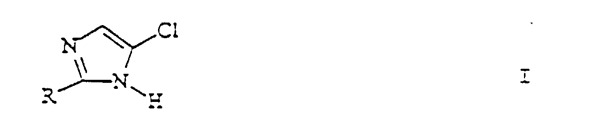 in which R has the stated meaning and the latter is finally reacted with phosphorus oxychloride or phosgene in the presence of N,N-dimethylformamide to yield the final product of the formula II.

2. Process according to claim 1, in which an alkali metal hydroxide or an alkali metal alkoxide is used as the base.

3. Process according to one of claims 1 or 2, in which the reaction to yield the 3,5-dihydroimidazol-4-one of the formula V is performed within a pH range between 7 and 12.

4. Process according to one of claims 1 to 3, in which the reactants for the reaction to yield the 3,5-dihydroimidazol-4-one of the formula V, namely glycine ester hydrohalide, imide acid ester and base, are reacted in a stoichiometric ratio of 1:1:1.

5. Process according to one of claims 1 to 4, in which the reaction temperature for the reaction to yield the 3,5-dihydroimidazol-4-one of the formula V is between -20°C and 50°C.

6. Process according to one of claims 1 to 5, in which the chlorination to yield the chloroimidazole of the formula I is performed with thionyl chloride or phosphorus oxychloride.

7. Process according to claim 6, in which the chlorination is performed with phosphorus oxychloride at a reaction temperature of between 20°C and 110°C.

8. Process according to one of claims 1 to 7, in which the reactants of the final stage, namely the 2-substituted 4-chloroimidazole of the formula I, the phosphorus oxychloride or the phosgene and the N,N-dimethylformamide are reacted in a molar ratio of 1:1:1 to 1:5:5.

9. Process according to one of claims 1 to 8, in which the reaction temperature in the final stage is between 50°C and 130°C.

## Revendications

1. Procédé pour la préparation des 5-chlorimidazoles 4-aldéhydes substitués sur la position 2, de la formule générale 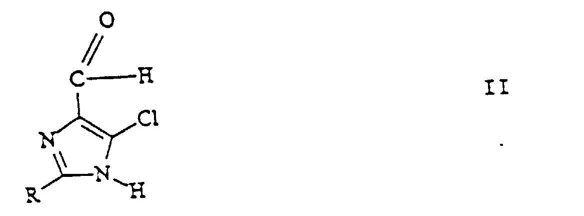 dans laquelle R signifie un groupe alkyle linéaire ou ramifié C₁-C₆, un groupe alcényle linéaire ou ramifié C₂-C₆, un groupe cyclopropyle, cyclobutyle, cyclopentyle ou cyclohexyle ou un groupe benzyle éventuellement substitué par halogène, nitro, amino et/ou alkyle ou un groupe phényle, caractérisé en ce que l'on transforme d'abord un hydrohalogénure d'ester de glycine de la formule générale 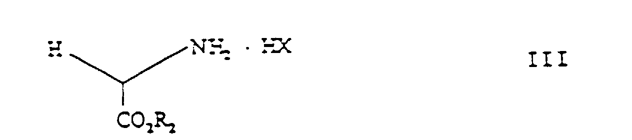 dans laquelle R₂ signifie un groupe alkyle et X signifie un atome d'halogène, avec un ester de l'acide imidique de la formule générale 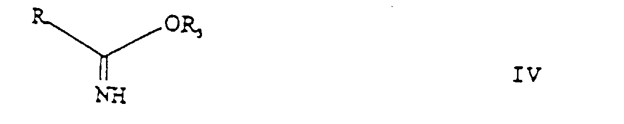 dans laquelle R a la signification ci-dessus et R₃ signifie un groupe alkyle, en présence d'une base, en la 3,5-dihydroimidazol-4-one correspondante de la formule générale 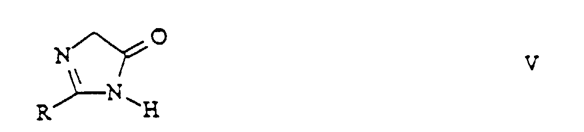 dans laquelle R a la signification ci-dessus, en ce que l'on chlore ensuite celle-ci en 4-chlorimidazole substitué en position 2, de la formule générale 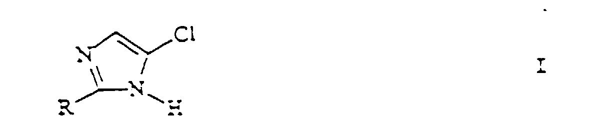 dans laquelle R a la signification ci-dessus, et en ce que l'on transforme enfin ce dernier avec de l'oxychlorure de phosphore ou du phosgène en présence de N,N-diméthylformamide en le produit final de la formule II.

2. Procédé selon la revendication 1, dans lequel comme base, on utilise un hydroxyde alcalin ou un alcoolat alcalin.

3. Procédé selon l'une des revendications 1 ou 2, dans lequel la transformation en 3,5-dihydro-imidazol-4-one de la formule V est conduite dans une plage de pH entre 7 et 12.

4. Procédé selon l'une des revendications 1 à 3, dans lequel les participants de la réaction pour la transformation en 3,5-dihydro-imidazol-4-one de la formule V, en l'occurrence l'hydrohalogénure d'ester de glycine, l'ester de l'acide imidique et la base, sont mis en réaction dans un rapport stoechiométrique de 1:1:1.

5. Procédé selon l'une des revendications 1 à 4, dans lequel la température réactionnelle pour la transformation en 3,5-dihydro-imidazol-4-one de la formule V, se situe entre -20°C et 50°C.

6. Procédé selon l'une des revendications 1 à 5, dans lequel la chloration en chlorimidazole de la formule I est conduite avec du chlorure de thionyle ou de l'oxychlorure de phosphore.

7. Procédé selon la revendication 6, dans lequel la chloration est conduite avec de l'oxychlorure de phosphore à une température réactionnelle entre 20°C et 110°C.

8. Procédé selon l'une des revendications 1 à 7, dans lequel les participants de la réaction de la dernière étape, en l'occurrence le 4-chlorimidazole substitué en position 2 de la formule I, l'oxychlorure de phosphore ou le phosgène et le N,N-diméthylformamide sont mis en réaction dans un rapport molaire de 1:1:1 jusqu'à 1:5:5.

9. Procédé selon l'une des revendications 1 à 8, dans lequel la température de réaction se situe entre 50°C et 130°C dans la dernière étape.
